Europäisches Patentamt

European Patent Office

Office européen des brevets

(11) Numéro de publication: **0 329 559 B1**

# FASCICULE DE BREVET EUROPEEN

(45) Date de publication de fascicule du brevet: **01.06.94**  (51) Int. Cl.⁵: **C12N 1/38**, C12N 9/58

(21) Numéro de dépôt: **89400441.5**

(22) Date de dépôt: **17.02.89**

(54) Procédé de l'obtention de la protéase d'Endothia parasitica.

(30) Priorité: **18.02.88 FR 8801934**

(43) Date de publication de la demande:
**23.08.89 Bulletin 89/34**

(45) Mention de la délivrance du brevet:
**01.06.94 Bulletin 94/22**

(84) Etats contractants désignés:
**AT BE CH DE ES FR GB GR IT LI LU NL SE**

(56) Documents cités:
**DE-A- 1 806 986**

**EUROPEAN BREWERY CONVENTION - PROCEEDINGS OF THE 20TH CONGRESS, Helsinki,1985, pages 419-425, IRL Press, Oxford, GB; J. OKSANEN et al.: "Microbial cellulase for improving filtrability of wort and beer"**

**P. DUPUY: "Utilisation des enzymes en technologie alimentaire", "Use of enzymes in food technology", Symposium International, Versailles, 5-7 mai 1982, pages 457-462, Lavoisier, Paris, FR; J.-C. VILLETTAZ et al.: "L'emploi des beta glucanese en oenologie"**

**CEREAL CHEMISTRY, vol. 63, no. 2, 1986, pages 124-130, American Association of Cereal Chemists, Inc., St Paul, MN, US; K.M. CHUNG et al: "Brewers' condensed solubles. III. Enzymatic hydrolysis, viscosity reduction, and fermentation"**

(73) Titulaire: **ELF SANOFI**
**32-34, rue Marbeuf**
**F-75008 Paris(FR)**

(72) Inventeur: **Couderc, René**
**6 chemin du Tricou Labège**
**F-31320 Castanet Tolosan(FR)**
Inventeur: **Eyssautier, Bruno**
**8, cité Auby**
**F-50500 Carentan(FR)**
Inventeur: **Laporte, Martine**
**58, rue Romain Rolland**
**F-31520 Ramonville Saint Agne(FR)**
Inventeur: **Planard, Marie-France**
**4, rue des Etiquenar**
**F-50500 Carentan(FR)**

(74) Mandataire: **Gillard, Marie-Louise et al**
**Cabinet Beau de Loménie**
**158, rue de l'Université**
**F-75340 Paris Cédex 07 (FR)**

**Description**

La présente invention concerne un procédé d'obtention de la protéase, coagulant le lait, sécrétée par Endothia parasitica.

La préparation de cette protéase est décrite dans le brevet français n° 1 401 474.

On a maintenant constaté que la viscosité du milieu de fermentation augmentait notablement au cours de la phase de production de la protéase rendant difficiles les opérations de purification et d'isolement et notamment l'ultrafiltration, et que la viscosité était notamment due à la présence d'un polysaccharide produit par le micro-organisme comme la protéase.

Le procédé selon l'invention consiste à introduire dans le milieu, pendant ou après la fermentation, une enzyme choisie parmi une β-1,3 glucanase, une β-1,3 β-1,6 glucanase ou une α-amylase ou un mélange desdites enzymes agissant sur le polysaccharide annexe produit par le micro-organisme.

L'enzyme peut être introduite dès le début de la fermentation, au cours de la fermentation ou seulement lors du traitement final du milieu de fermentation, avant la filtration de la biomasse ou au cours de la concentration du filtrat par ultrafiltration ou microfiltration.

On peut aussi introduire dans le milieu différentes enzymes, simultanément ou séparément, à différentes étapes du procédé.

Malgré la présence de protéase dans le milieu, l'enzyme agit sur le polysaccharide et diminue la viscosité de ce milieu, que ce soit en cours de fermentation ou après l'arrêt de celle-ci.

Dans le procédé de production de protéase coagulante par fermentation d'Endothia parasitica, on peut utiliser des glucanases telles que la bêta-1,3 glucanase de basidiomycètes décrite dans US 3 423 288, les bêta-1,3 bêta-1,6 glucanases, comme celle commercialisée par NOVO sous la marque Glucanex ou celle commercialisée par Gist-Brocades sous la dénomination Rapidase GL 150 ou encore des alpha-amylases fongiques, comme la Fungamyl commercialisée par NOVO, ou l'amylase P 200 commercialisée par Gist-Brocades.

On n'introduira en général l'enzyme dans le milieu de fermentation qu'après une durée de culture supérieure à 50 % de la durée totale prévue pour une fermentation sans enzyme et de préférence à 75 % ; dans ces conditions, la viscosité du milieu reste limitée et la production de protéase n'est pas diminuée ou peut même être améliorée ; on constate en effet que la viscosité du moût en fin d'opération est nettement inférieure à celle d'une fermentation sans enzyme, de 2 à 4 fois, sans diminution de la biomasse.

Le filtrat, obtenu dans ce cas après séparation des cellules, est moins visqueux et peut être concentré par ultrafiltration dans un dispositif classique jusqu'à une concentration supérieure à 30 g/l de protéase et même jusqu'à 40 g/l, alors qu'après une fermentation classique on n'atteint que 10 g/l environ ; enfin, la filtration finale sur membrane de faible porosité est facilitée.

Selon un autre aspect, le procédé selon l'invention consiste à traiter les moûts de fermentation avant leur filtration, pendant 1 à 5 heures, avec 50 mg/l à 1 g/l d'une préparation enzymatique active sur le polysaccharide ; la viscosité du moût est diminuée de 2 à 5 fois ainsi que le temps de filtration nécessaire à la séparation de la biomasse du milieu contenant le produit recherché.

Selon un dernier aspect, le procédé selon l'invention consiste à traiter le filtrat obtenu après séparation des cellules, par une enzyme hydrolysant le polysaccharide annexe au cours de la concentration de ce filtrat par ultrafiltration, ce qui permet d'obtenir une solution présentant une forte concentration en produit recherché, lorsque celui-ci ne peut être isolé directement par précipitation ou évaporation du solvant sans dénaturation ; dans ce cas, on préfère alors utiliser une alpha-amylase.

Dans ce qui suit, on décrit des exemples de mise en oeuvre de l'invention, pour la fermentation d'Endothia parasitica.

Les inoculums sont préparés à partir de spores congelées d'Endothia parasitica, de la souche ATCC 14729, par culture à 28°C dans un milieu aqueux stérilisé contenant du glucose, de la farine de soja, des sels minéraux et des extraits autolytiques de levures.

La production est effectuée à 28°C dans un milieu aqueux stérile du même type que le précédent.

Les viscosités sont mesurées au viscosimètre Brookfield ; les activités coagulantes ont été déterminées par la méthode préconisée par la Fédération Internationale de Laiterie et publiée dans le Journal Officiel de la République Française du 20 mars 1981.

**EXEMPLE 1 Traitements enzymatiques en cours de fermentation**

Les essais ont été réalisés avec le Glucanex ou l'amylase P 200 introduits à différents moments de la fermentation, la durée totale de celle-ci étant voisine de 90 heures. L'amylase P 200 contient une alpha-amylase fongique d'Aspergillus oryzae et se présente sous forme d'une poudre, de titre FAU : 4540/g, un

2

FAU correspondant à la quantité d'enzyme hydrolysant 5,26 g d'amidon soluble en 1 heure, à 37°C et pH 4,7.

Le Glucanex est une poudre contenant une enzyme sécrétée par une souche de Trichoderma ; elle présente 300 unités bêta-glucanase/gramme.

Les quantités d'enzyme ajoutées, les temps d'addition ainsi que la viscosité du milieu de fermentation et l'activité coagulante du concentrat sont indiqués dans le tableau I.

TABLEAU I

| Essai N° | Enzyme | Concentration de l'enzyme (mg/l) | Heures d'addition | Viscosités du milieu (mPa.s) | | | | | Activité coagulante du concentrat g/l | Viscosité du concentrat (mPa.s) |
|---|---|---|---|---|---|---|---|---|---|---|
| | | | | 40 h | 47 h | 64 h | 72 h | 88 h | | |
| 1 | Glucanex | 20 | 0 | | | | 1000 | 1140 | 15 | |
| 2 | " | 20 | 42 | | | | 950 | 680 | 28 | |
| 3 | Témoin | 0 | | 1900 | 2600 | 3750 | 3700 | 2200 | 8,2 | 93 |
| 4 | Glucanex | 20 | 65 | 1900 | 2500 | 3050 | 1600 | 890 | 30 | 8 |
| 5 | " | 50 | 65 | | 2250 | 2700 | 750 | 420 | 41 | 9 |
| 6 | " | 100 | 65 | | 2250 | 2700 | 400 | 380 | 43 | 9 |
| 7 | Témoin | 0 | | 1100 | 1700 | - | 1900 | 1000 | | |
| 8 | Glucanex | 10 | 45 et 68 | 950 | 1300 | - | 1100 | 650 | | |
| 9 | Témoin | 0 | | 720 | 920 | 640 | - | 800 | | |
| 10 | Glucanex | 200 | 23 | 240 | 240 | 106 | - | 400 | | |
| 11 | Amylase P200 | 20 | 45 et 65 | 1300 | 800 | 820 | 1000 | 850 | | |
| 12 | " | 40 | 65 | 1300 | 1250 | 1200 | 900 | | | |
| 13 | Témoin | 0 | 0 | 1500 | 1900 | 2200 | 2800 | 2100 | | |
| 14 | Glucanex | 42 | 72 | 1900 | 1800 | 1600 | 2700 | 900 | | |

3

Les moûts des essais 13 et 14 ont été traités après 90 heures de fermentation : filtration sur toile pour séparer le mycélium, puis concentration du filtrat par ultrafiltration avec une membrane perméable aux composés de masse moléculaire inférieure à 10 000 environ.

Les résultats obtenus figurent dans le tableau II.

L'essai 13 a été effectué de façon classique sans addition d'enzyme ; dans l'essai 14, on a ajouté 40 mg de Glucanex par litre de moût après 72 heures de fermentation.

TABLEAU II

| Essai n° | Viscosité Moût (mPa.s) | Poids du moût | Durée filtration | Poids mycélium | Viscosité du filtrat | Activité coagulante du concentrat | Viscosité du concentrat |
|---|---|---|---|---|---|---|---|
| 13 | 1600 | 158 kg | 24 min | 31 kg | 5 | 10 g/l | 120 mPa.s |
| 14 | 900 | 142 kg | 19 min | 30,5 kg | 4 | 38 g/l | 120 mPa.s |

## EXEMPLE 2 Traitements enzymatiques du moût en fin de fermentation

Les moûts ont été traités après 88 heures de culture ; ils contenaient environ 7,5 g/kg de polysaccharide et avaient une viscosité de 1000 mPa.s. On a ajouté 200 mg/l de différentes préparations enzymatiques en poudre ou 10 ml/l de préparations enzymatiques liquides.

Les résultats obtenus pour différents temps de contact à température ambiante figurent dans le tableau III.

TABLEAU III

| Préparation enzymatique | Fournisseur | Activité enzymatique | Viscosité du moût (mPa.s) après | | | |
|---|---|---|---|---|---|---|
| | | | 0,5h | 1h | 2h | 5h |
| Hémicellulase Reg 2 | Gist-Brocades | Pectinase + galactanase + galactomannase | 920 | 770 | 520 | 550 |
| Fungamyl | Novo | Alpha-amylase | 690 | 550 | 550 | 450 |
| Amylase P 200 | Gist-Brocades | Alpha-amylase | 850 | 600 | 680 | 550 |
| Glucanex | Novo | Bêta-1,3 bêta-1,6 glucanase | 650 | 420 | 280 | 150 |
| Rapidase GL 150 | Gist-Brocades | Bêta-1,3 bêta-1,6 glucanase | 850 | 600 | 520 | 480 |
| Finizym | Novo | Bêta-1,3 bêta-1,4 glucanase | 920 | 900 | 880 | 850 |

## EXEMPLE 3 Traitements enzymatiques du filtrat de fermentation, après séparation du mycélium

L'addition d'enzyme a été effectuée avant le début de l'ultrafiltration. Le Fungamyl est une préparation liquide d'alpha-amylase fongique de titre 800 FAU/g ; le Maxamyl et le BAIF, commercialisés par Gist-Brocades, sont des amylases bactériennes.

Le tableau IV indique la chute de la viscosité au cours du temps d'un filtrat préconcentré, dans lequel ont été introduites différentes enzymes, en quantités variables.

Le filtrat, préconcentré par ultrafiltration, ayant un pH de 4,3, avait une viscosité de 70 mPa.s ; les quantités des préparations enzymatiques sont exprimées en g pour 100 g de protéase dans le filtrat préconcentré.

4

TABLEAU IV

| Préparation enzymatique | Quantité g/100 g | Viscosité (mPa.s) après | | | | |
|---|---|---|---|---|---|---|
| | | 25 min | 50 min | 100 min | 150 min | 175 min |
| Maxamyl | 4,76 | 58 | 46 | 38 | 32 | |
| BAIF | 3,8 | 46 | 32 | 26 | 24 | |
| Amylase P 200 | 4,76 | 5 | 5 | 5 | 5 | 5 |
| | 0,95 | 7,5 | 5 | 5 | 5 | 5 |
| | 0,095 | 22 | 18 | 16 | 15 | 14 |
| Fungamyl | 4,76 | 10 | 10 | 10 | | |
| | 0,095 | 28 | 20 | 16 | | |
| Glucanex | 0,95 | 63 | 62 | 63 | 62 | |
| Dextranase Novo | 4,76 | 70 | 68 | | 65 | |
| Pectinase SP 249 Novo | 4,76 | 70 | 68 | | 65 | |

**EXEMPLE 4 Traitements enzymatiques au cours de la concentration du filtrat**

L'ensemble du procédé a été effectué de façon classique : fermentation, séparation du ferment par filtration et concentration du filtrat par ultrafiltration.

Cette dernière opération a été arrêtée lorsque le débit est devenu pratiquement nul. Le concentrat, de pH 3,9, contenait alors 9 g/l de protéase.

On a introduit 40 mg d'amylase P 200 ou 40 mg de Fungamyl pour 10 kg de concentrat et repris l'ultrafiltration après avoir laissé les mélanges 2h15 à température ambiante.

Après 45 minutes de concentration, on a obtenu un concentrat dont la concentration en protéase était de 45 g/l dans le cas du traitement à l'amylase P 200 et de 42 g/l dans le cas du traitement au Fungamyl.

**Revendications**

1. Procédé d'obtention de la protéase d'Endothia parasitica, caractérisé en ce que l'on introduit dans les milieux, pendant ou après la fermentation, une enzyme choisie parmi une $\beta$-1,3 glucanase, une $\beta$-1,3 $\beta$-1,6 glucanase ou une $\alpha$-amylase ou un mélange desdites enzymes agissant sur le polysaccharide viscosifiant annexe, produit par le champignon.

2. Procédé selon la revendication 1, caractérisé en ce que l'enzyme est introduite dans le milieu pendant la fermentation.

3. Procédé selon la revendication 1, caractérisé en ce que l'enzyme est introduite dans le moût en fin de fermentation avant la séparation des cellules.

4. Procédé selon la revendication 1, caractérisé en ce que l'enzyme est introduite dans le milieu, après séparation des cellules, avant sa concentration par ultrafiltration ou micro-filtration.

5. Procédé selon la revendication 1, caractérisé en ce que l'enzyme est introduite dans le milieu au cours de la concentration par ultrafiltration.

6. Procédé selon la revendications 1 caractérisé en ce que l'enzyme est une $\beta$-1,3 $\beta$-1,6 glucanase introduite dans le milieu pendant ou à la fin de la fermentation.

7. Procédé selon la revendication 1, caractérisé en ce que l'enzyme est une $\alpha$-amylase introduite dans le milieu au cours de sa concentration.

Claims

1. Process for obtaining protease from Endothia parasitica, characterized in that an enzyme selected from a β-1,3 glucanase, a β-1,3 β-1,6 glucanase or an α-amylase or a mixture of said enzymes acting on the subsidiary viscosity-inducing polysaccharide produced by the fungus, is introduced in the medium, during or after fermentation.

2. Process according to claim 1, characterized in that the enzyme is introduced in the medium during the fermentation.

3. Process according to claim 1, characterized in that the enzyme is introduced in the broth at the end of the fermentation before the separation of the cells.

4. Process according to claim 1, characterized in that the enzyme is introduced in the medium, after separation of the cells, before its concentration by ultra-filtration or micro-filtration.

5. Process according to claim 1, characterized in that the enzyme is introduced in the medium during the concentration by ultra-filtration.

6. Process according to claim 1, characterized in that the enzyme is a β-1,3 β-1,6 glucanase, introduced in the medium during or at the end of the fermentation.

7. A Process according to claim 1, characterized in that the enzyme is an α-amylase introduced in the medium during its concentration.

Patentansprüche

1. Verfahren zur Gewinnung der Protease von Endothia parasitica,
dadurch gekennzeichnet,
daß während oder nach der Fermentation ein unter β-1,3-Glucanasen, β-1,3, β-1,6-Glucanasen und α-Amylasen ausgewähltes Enzym oder ein Gemisch dieser Enzyme in die Medien hineingegeben wird, die auf das durch den Pilz als Nebenprodukt gebildete und die Viskosität erhöhende Polysaccharid einwirken.

2. Verfahren nach Anspruch 1, dadurch gekennzeichnet, daß das Enzym während der Fermentation in das Medium gegeben wird.

3. Verfahren nach Anspruch 1, dadurch gekennzeichnet, daß das Enzym nach der Fermentation und vor der Abtrennung der Zellen in die Kulturbrühe gegeben wird.

4. Verfahren nach Anspruch 1, dadurch gekennzeichnet, daß das Enzym nach der Abtrennung der Zellen und vor der Aufkonzentrierung des Mediums durch Ultrafiltration oder Mikrofiltration in das Medium gegeben wird.

5. Verfahren nach Anspruch 1, dadurch gekennzeichnet, daß das Enzym während der Aufkonzentrierung durch Ultrafiltration in das Medium gegeben wird.

6. Verfahren nach Anspruch 1, dadurch gekennzeichnet, daß das Enzym eine β-1,3, β-1,6-Glucanase ist, die während oder nach der Fermentation in das Medium gegeben wird.

7. Verfahren nach Anspruch 1, dadurch gekennzeichnet, daß das Enzym eine α-Amylase ist, die während der Aufkonzentrierung in das Medium gegeben wird.